(19) Europäisches Patentamt

European Patent Office

Office européen des brevets

(11) Publication number: **0 545 352 A1**

## (12) EUROPEAN PATENT APPLICATION

(21) Application number: **92120475.6**

(22) Date of filing: **01.12.92**

(51) Int. Cl.5: **C07K 9/00**, C12P 21/00, A61K 35/74

(30) Priority: **03.12.91 IT MI913238**

(43) Date of publication of application: **09.06.93 Bulletin 93/23**

(84) Designated Contracting States: **AT BE CH DE DK ES FR GB GR IE IT LI LU NL PT SE**

(71) Applicant: **Fichera, Guiseppe Viale Artale Alagona, 75 I-95126 Catania(IT)**

(72) Inventor: **Fichera, Guiseppe Viale Artale Alagona, 75 I-95126 Catania(IT)**

(74) Representative: **Gervasi, Gemma et al NOTARBARTOLO & GERVASI Srl Viale Bianca Maria 33 I-20122 Milan (IT)**

(54) **Pharmaceutical compositions containing Lactobacillus casei peptidoglycan.**

(57) Pharmaceutical compositions containing pharmacologically active amounts of Lactobacillus casei peptidoglycan suitable for use in anti-tumor therapy.

EP 0 545 352 A1

Prior Art

In recent years the results of numerous investigations were published demonstrating that Lactobacillus casei, a gram-positive non pathogenic microorganism, shows an anti-tumor activity, deriving from immunostimulations, against experimental and human malignant tumors, as described for instance by Kato I, Kobayashi S. Yokokura T, Mutai M (1981): Antitumor activity of Lactobacillus casei in mice, Gann 72:517 and by Matsuzaki T., Yokokura T., Mutai M (1988). Antitumor effect of intrapleural administration of Lactobacillus casei in mice, Cancer Immunol. Immunother. 26:209.

It was also proved that feeding by products fermented by Lactobacillus casei brings about an enancement of the anti-tumor activity in experiment animals, as described e.g. by Friend BA, Farmer RE, Shahani KM (1982): Effect of feeding and intraperitoneal implantation of yoghurt culture cells on Ehrlich ascites tumor, Milchwissenschaft 37:708.

This activity was considered to be host-mediated in view of the fact that no direct toxicity was found for tumoral cells in vitro as shown, e.g., by Kato I, et al. (l.c.).

Summary

It was now found that surprisingly higher effects are obtained in the inhibition of tumoral cell activity by treatment with a suitably prepared peptidoglycan of Lactobacillus casei, which operates as an inhibitor of the tumor cell activity with a direct toxicity mechanism.

The present invention refers therefore to the use of said peptidoglycan for the preparation of pharmaceutical compositions suitable for anti-tumor therapy.

Detailed description of the Invention

The characteristics of the peptidoglycan of Lactobacillus casei and the advantages of its use in anti-tumor therapy according to the present invention will be further evidenced in the following detailed description.

We have found that the peptidoglycan derived from Lactobacillus casei, prepared as described further below, has a high toxic anti-tumor direct activity against many lines of tumor cells.

This activity was evaluated with the aid of the following methods:
release of $^{51}$Cr from marked target cells, and inhibition of the succinate dehydrogenase (SDH) activity (enzymatic test apt to detect the cell death or survival).

The peptidoglycan was prepared as follows:
Lactobacillus casei ATCC 25180 supplied by Nestle Co., Orbe, Switzerland, was cultivated in a MRS medium for 20 hrs at 30°C, and the cells centrifuged out, washed with Ringer solution pasteurized for 10 min. and lyophilized.

From the thus prepared cells, the peptidoglycan was obtained as follows:
Lactobacillus casei cellular walls were obtained by mechanical rupture of said cells in a homogeneizer and successive washing comprising repeated centrifugations. They were then treated with 10% trichloroacetic acid for eight hours in order to extract teichoic acid.

From the thus obtained cellular walls the peptidoglycan was solubilized in water by repeated treatments in a ultrasonic disintegrator at 20-22 Kc/sec at 4°C.

After this, the insoluble residue was removed by centrifugation at 30,000 t for 30 min. and the obtained solution brought to pH 7.2 and lyophilized.

The peptidoglycan molecule consists of a network of polysaccharide chains covalently bound to each other by short polypeptide bridges bound to each muramic acid residue. The polysaccharide structure on its turn consists of a ripetitive disaccharide unit of N-acetylglucosamine and acetylmuramic acid with a $\beta$ bond (1-4).

As targets the following cells were experimented: YAC-1 (from murine lymphoma), K562 (from human erithroleucemia), P815 (from murine mastocytoma), EATC (from Ehrlich's ascite tumor), KB (from human oral epidermoid carcinoma).

The YAC-1, K562 and P815 cells were cultivated in a RPMI 1640 medium supplemented by 10% fetal bovine serum (FBS).

The EATC and KB cells were cultivated in a DMEM medium with 10% FBS. 6 For the $^{51}$Cr release text, the target cells ($10$-$20 \times 10^6$) were incubated with 100 $\mu$Ci $Na_2{}^{51}CrO_4$ in one ml RPMI at 37°C for 60 minutes under intermittent stirring, washed 3 times with 10% FBS RPMI and finally diluted with this medium at a concentration of $1 \times 10^5$ cell/ml.

EP 0 545 352 A1

In the test, 100 $\mu$l of this target cell suspension are added to the U-bottom wells of a micro-titration plate and to each well 100 $\mu$l of RPMI containing the peptidoglycan at the desired concentration are added.

The plates are incubated for 4 hrs at 37°C in a moist atmosphere containing 5% $CO_2$.

At the end of this incubation period, the plates are centrifuged at 400 t for 10 minutes.

From the surnatant of each well 100 $\mu$l aliquots are taken and the radioactivity is measured with the aid of a gamma counter.

The specific lysis percent is figured as follows:

$$\frac{\text{experimental release (cpm)} - \text{spontaneous release (cpm)}}{\text{total release (cpm)} - \text{spontaneous release (cpm)}} \times 100$$

Total release refers to the counts per minute obtained after addition of 100 $\mu$l Triton X-100 solution, and spontaneous release to the counts per minute due to target cells in the presence of RPMI medium only.

Spontaneous release does not surpass 10% of total release.

The method for determining succinate dehydrogenase (SDH) is based on the scission of MTT tetrazolium salt [3-(4,5-dimethylthiazol-2-yl)-2,5-diphenyl tetrazolium bromide] by the mitochondrial dehydrogenase succinate enzyme to form a blue colored product (formazan).

As the conversion takes place only in live cells, the amount of produced formazan is proportional to the number of live cells presents.

The cells are grown in a medium consisting of serum containing RPMI; however, for the test this medium is removed and the cells are suspended in a new medium (RPMI-PR$^-$) from which the phenol red indicator and the serum are omitted.

The final tested cell concentration is $10^4$/ml or $10^5$/ml and to each well of a flat bottom microtitration plate 50 $\mu$l of cell suspension are added.

50 $\mu$l peptidoglycan in the desired concentration are then added, the plate incubated for 1 hr at 37°C, centrifuged (800 t, 10 min.), the supernatant removed, and to the cells in each well 50 $\mu$l of a MTT solution in a RPMI-PR$^-$ medium are added. After incubation for 3 hrs at 37°C, the plate is centrifuged again (800 t, 10 min.) and the non transformed MTT removed.

Finally 50 $\mu$l isopropanol are added to each well and the plate is vigorously agitated to completely dissolve the formazan blue. The optical density is measured at a wavelength of 540 nm for the test and of 690 nm for the reference.

The tests on treating the tumoral cellular K562, YAC-1, P815, EATC and KB with the Lactobacillus casei peptidoglycan have shown a considerable cytotoxic activity, as reported in Table 1, in which the determination of test 1 was carried out with the method of $^{51}$Cr release, while in the other tests the SDH activity inhibition method was employed.

Table 1

| Cytotoxic % activity of Lactobacillus casei peptidoglycan | | |
| --- | --- | --- |
| Test N. | Cell lines | Cytotoxic activity % |
| 1 | K562 | 21.1 ± 6.34[6] |
| 2 | YAC-1 | 26.5 ± 6.34[6] |
| 3 | K562 | 18.0 ± 8.77[6] |
| 4 | P815 | 32.3 ± 7.65[6] |
| 5 | EATC | 13.6 ± 4.72[12] |
| 6 | KB | 37.4 ± 6.01[8] |

(n) number of determinations.

In these tests, the number of cells was 1x$10^5$/ml and the peptidoglycan concentration 40 $\mu$g/ml.

In parallel tests using as toxicity agent whole Lactobacillus casei killed by heat, at a 60 $\mu$g/ml concentration, a decidedly lower percent toxicity was found.

Further confirmation of these results was obtained by cytotoxicity tests on YAC-1 lymphoma at increasing whole Lactobacillus casei and peptidoglycan concentrations.

3

EP 0 545 352 A1

These tests were performed at a tumoral cell concentration of $8.10^5$/ml, at 37°C for 60 minutes.

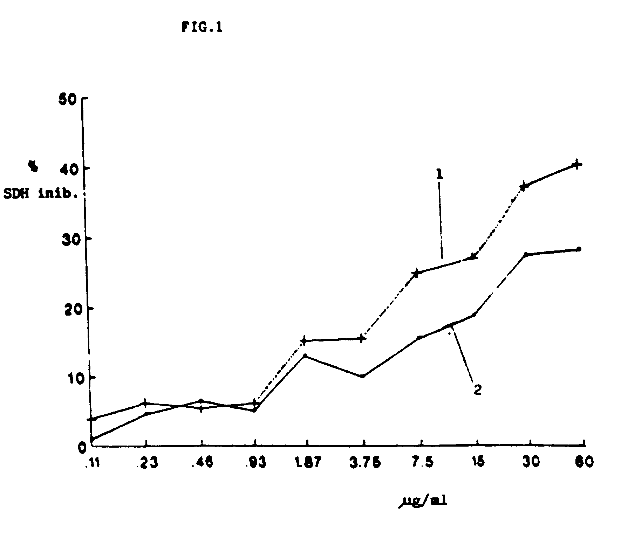

The results of these tests are reported in Fig. 1, in which line 1 refers to the peptidoglycan treatment and line 2 to the Lactobacillus casei treatment; in the ordinates the SDH percent inhibition and in the abscissae the treatment agent concentration are reported.

A further important aspect of the present invention consists in the fact that the peptidoglycan acts on tumor cells after a few minutes from the addition, as shown by the increase of the $^{51}$Cr release and by the SDH activity inhibition which are in fact observed a few minutes after the peptidoglycan addition to the tumor cells.

This suggests an activity mechanism based on the direct toxicity of peptidoglycan vis-a-vis the tumor cells, while the information available from the prior technique on the Lactobacillus casei activity refer to a mechanism of cell defense stimulation which implies nuch longer times, even of a few days.

Thanks to the above reported characteristics, the Lactobacillus casei peptidoglycan may be usefully employed for preparing pharmaceutical compositions suitable to the therapy of human tumor pathologies. Said compositions contain pharmacologically active amounts of said peptidoglycan plus compatible diluents and excipients normally employed in pharmaceutical technique.

Preferably, the peptidoglycan, dissolved in normal biocompatible buffered solvents, is formulated with liposomes consisting of cholesterol and of phosphatidyl-choline and phosphatidyl-inositol derivatives.

## Claims

1. Lactobacillus casei peptidoglycan obtained by mechanical rupture of Lactobacillus casei cells followed by treatment of the obtained cellular walls with trichloroacetic acid and solubilization in water through a ultrasonic disintegration treatment.

2. The use, for the preparation of pharmaceutical compositions suitable for the therapy of tumor pathologies, of Lactobacillus casei peptidoglycan according to claim 1.

3. The use according to claim 2, characterized in that said compositions contain pharmacologically active amounts of Lactobacillus casei peptidoglycan plus compatible diluents and excipients commonly employed in the pharmaceutical technique.

4. The use according to claim 2, characterized in that said compositions contain pharmacologically active amounts of Lactobacillus casei peptidoglycan dissolved in a compatible solvent and formulated with liposomes consisting of cholesterol and of phosphatidylcholine and phosphatidylinositol derivatives.

5. The use in the therapy of tumor pathologies of Lactobacillus casei peptidoglycan obtained by mechanical rupture of Lactobacillus casei cells followed by treatment of the cellular walls with trichloroacetic acid and solubilization in water through a ultrasonic disintegration treatment.

4

FIG.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | PATENT ABSTRACTS OF JAPAN vol. 12, no. 479 (C-552)(3326) 14 December 1988 & JP-A-63 196 521 ( YAKULT HONSHA CO LTD ) 15 August 1988 * abstract * | 1,2,5 | C07K9/00 C12P21/00 A61K35/74 |
| X | CHEMICAL ABSTRACTS, vol. 113, no. 7, 13 August 1990, Columbus, Ohio, US; abstract no. 52183h, MATSUZAKI, TAKESHI ET AL. 'Antitumor effect of polysaccharide-peptidoglycan complex (PS-PG) of Lactobacillus casei YIT 9018 on Meth A.' page 25 ;column R ; & YAKURI TO CHIRYO vol. 18, no. 1, 1990, pages 51 - 57 | 1,2,5 | |
| X | BIOCHEMISTRY. vol. 8, no. 9, September 1969, EASTON, PA US pages 3567 - 3577 K.D. HUNGERER ET AL. 'Structure of the Cell Wall Peptidoglycab of Lactobacillus casei RO94' * page 3567, left column, paragraph 1 - page 3568, left column, paragraph 3 * | 1 | TECHNICAL FIELDS SEARCHED (Int. Cl.5 ) C07K A61K C12P |
| X | EP-A-0 406 087 (KABUSHIKI KAISHA YAKULT HONSHA) * page 2, line 55 - page 5, line 10 * | 1 | |
| A | WO-A-9 116 347 (IMMUNOTHERAPEUTICS, INC.) * page 3, line 1 - page 4, line 3 * * page 7, line 36 - page 8, line 20 * | 1-5 | |

The present search report has been drawn up for all claims

| Place of search | Date of completion of the search | Examiner |
|---|---|---|
| THE HAGUE | 24 MARCH 1993 | MONTERO LOPEZ B. |